# EUROPEAN PATENT APPLICATION

(11) **EP 4 049 655 A1**
(43) Date of publication of application: **31.08.2022**
(21) Application number: 20879456.0
(22) Date of filing: 14.10.2020
(51) Int. Cl.: A61K 9/70, A61K 31/19, A61K 31/433, A61K 47/06, A61K 47/10, A61K 47/14, A61K 47/32, A61K 47/34, A61P 21/02, B32B 5/02, B32B 27/00

(54) **LAYERED TRANSDERMAL PATCH**

(30) Priority: 21.10.2019 JP 2019191677
(71) Applicant: MEDRx Co., Ltd., Higashikagawa-shi Kagawa 769-2712 (JP)
(72) Inventor: MIWA Yasushi, Higashikagawa-shi Kagawa 769-2712 (JP); HAMAMOTO Hidetoshi, Higashikagawa-shi Kagawa 769-2712 (JP)
(74) Representative: Bertsch, Florian Oliver
(86) International application number: PCT/JP2020/038707
(87) International publication number: WO 2021/079795

(57) **Abstract**

It is an object of the present invention to provide a new patch which is excellent in drug retention and adhesiveness to skin.

The present invention, for example, can include a laminated type patch A, comprising a release layer 1, a drug layer 2, a drug support layer 3 having elasticity, an adhesive layer 4, and an adhesive support layer 5 laminated in this order, wherein the outer edges of the release layer, the adhesive layer, and the adhesive support layer are all outside the outer edges of both the drug layer and the drug support layer; wherein the portion surrounded by the outer edges of the drug layer and the drug support layer, and the inner sides of the release layer and the adhesive layer has a space; and wherein the cross-sectional area of the space is 0.3 mm² or more, at least when cut along the longitudinal centerline and the transverse centerline on the plane surfaces of the drug layer and the drug support layer.

The laminated type patch A according to the present invention is excellent in retention of a drug and adhesiveness to the skin.

## Description

### TECHNICAL FIELD

### (Cross-Reference to Related Applications)

This application claims the benefit of Japanese Patent Application No. 2019-191677, filed on October 21, 2019 with the Japanese Patent Office. The Japanese application is hereby incorporated by reference for all purposes as if the entirety of its application documents (specification, claims, drawings, and abstract) were expressly set forth herein.

The present invention relates to a laminated type patch. The present invention relates particularly to a laminated type patch having a space, which is excellent in drug retention and adhesiveness to the skin.

### BACKGROUND OF THE INVENTION

From the past, a patch has been known as a formulation form for an efficient and long-lasting transdermal administration of a drug. As an attempt to improve the adhesiveness of a patch, a cover including an adhesive layer is provided so as to surround a plaster portion containing a drug. For example, a patch with a cover material, containing a drug having a specific dissolution parameter and a base (Patent Document 1), and a patch with a laminated structure, containing a buffer material between the adhesive layer and the cover layer (Patent Document 2).

### PRIOR ART

### PATENT DOCUMENT

Patent Document 1: WO 2015/087927, A
Patent Document 2: WO 2015/133329, A

### SUMMARY OF THE INVENTION

### PROBLEM TO BE SOLVED BY THE INVENTION

Even if a cover layer is provided on a patch as in the above-mentioned prior inventions, an ingredient in a drug layer or in a plaster may leak from the inside of the drug layer to the cover layer, and the drug effect may decrease. Therefore, it has been desired to develop a patch holding a drug in the plaster portion for a long period of time to prevent the ingredient in the drug layer from leaking out, and having high adhesiveness to the skin.

The present invention chiefly aims to provide a novel patch which is excellent in drug retention and adhesiveness to the skin.

### MEANS FOR SOLVING THE PROBLEMS

In order to solve the above-mentioned problem, the present inventors have investigated, for example, a structure having a release layer and a cover layer and a material of a support portion, in a patch product having what is called a cover layer as disclosed in Patent Document 2.

As a result, the present inventors have found that by providing a slight space in an adhered portion between a release layer and a cover layer, it is possible to suppress a drug from being transferred to a cover layer, and to improve the adhesiveness to the skin, and also have found that it is possible to efficiently adhere a drug layer to the skin by using a material having elasticity in a support portion of a drug layer, and the present invention was completed.

The present invention can include, for example, the following embodiments.
[1] A laminated type patch, comprising a release layer, a drug layer, a drug support layer having elasticity, an adhesive layer, and an adhesive support layer laminated in this order,
   wherein the outer edges of the release layer, the adhesive layer, and the adhesive support layer are all outside the outer edges of both the drug layer and the drug support layer;
   wherein the portion surrounded by the outer edges of the drug layer and the drug support layer, and the inner sides of the release layer and the adhesive layer has a space; and
   wherein the cross-sectional area of the space is 0.3 mm² or more, at least when cut along the longitudinal centerline and the transverse centerline on the plane surfaces of the drug layer and the drug support layer.
[2] The laminated type patch according to the [1] mentioned above, wherein the cross-sectional area of the space is 0.3 mm² or more on average, when cut in the longitudinal direction and the transverse direction including the longitudinal centerline and the transverse centerline on the plane surfaces of the drug layer and the drug support layer.
[3] The laminated type patch according to the [1] or [2] mentioned above, wherein the average thickness of the drug support layer is 0.4 mm or more.
[4] The laminated type patch according to any one of the [1] to [3] mentioned above, wherein the drug support layer consists of one or more kinds selected from the group consisting of a nonwoven fabric, a woven fabric, and an elastomer film.
[5] The laminated type patch according to the [4] mentioned above, wherein the drug support layer consists of a nonwoven fabric.
[6] The laminated type patch according to the [5] mentioned above, wherein the weight per unit of the nonwoven fabric is within the range of 50 to 160 g/m².
[7] The laminated type patch according to the [5] or [6] mentioned above, wherein the nonwoven fabric consists of a polyester resin.
[8] The laminated type patch according to any one of the [1] to [7] mentioned above, wherein the adhesive support layer has elasticity.
[9] The laminated type patch according to any one of the [1] to [8] mentioned above, wherein the drug layer contains a colored drug.
[10] The laminated type patch according to any one of the [1] to [9] mentioned above, wherein the drug layer contains one or more solvents selected from the group consisting of a hydrocarbon, a polyhydric alcohol, and an ester.
[11] The laminated type patch according to any one of the [1] to [10] mentioned above, wherein the adhesive layer contains one or more polymers selected from the group consisting of an acrylic polymer, a rubber-based polymer, and a silicone-based polymer.
[12] The laminated type patch according to any one of the [1] to [11] mentioned above, wherein the release layer is treated with a silicone.
[13] The laminated type patch of any one of the [1] to [12] mentioned above, wherein the plane area of the drug layer is within the range of 90% to 100% of the plane area of the drug support layer.
[14] The laminated type patch according to any one of the [1] to [13] mentioned above, wherein the plane area of the drug layer is within the range of 20% to 90% of the plane area of the adhesive layer.
[15] The laminated type patch according to any one of the [1] to [14] mentioned above, wherein the plane area of the drug support layer is within the range of 20% to 90% of the plane area of the adhesive layer.
[16] The laminated type patch according to any one of the [1] to [15] mentioned above, wherein the average value of a distance from an outer edge of the drug support layer to an outer edge of the adhesive layer is within the range of 0.5 cm to 3 cm.
[17] The laminated type patch according to any one of the [1] to [16] mentioned above, wherein the plane area of the adhesive layer is within the range of 90% to 100% of the plane area of the adhesive support layer.

### EFFECT OF THE INVENTION

The laminated type patch according to the present invention is excellent in drug retention and adhesiveness to the skin.

The problem of a patch is that a required amount of a drug needs to be absorbed continuously from the skin. For that, it is necessary to keep the required amount of a drug in contact for a necessary time. However, the amount of absorption fluctuates due to diffusion of a drug in a patch and slippage of a patch itself. The present invention enables quantitative administration of a drug, since retention of the drug in a drug plaster having a predetermined size for a long period of time is possible, and adhesiveness to the skin is excellent.

### BRIEF DESCRIPTION OF THE DRAWINGS

Figure 1 shows an X-X cross-sectional view of a laminated type patch according to the present invention.
Figure 2 shows a plan view of a laminated type patch according to the present invention.

### EMBODIMENT FOR CARRYING OUT THE PRESENT INVENTION

### 1 Patch according to the present invention

A laminated type patch according to the present invention (hereinafter, referred to as "the present invention patch ") is a patch, comprising a release layer, a drug layer, a drug support layer having elasticity, an adhesive layer, and an adhesive support layer laminated in this order, wherein the outer edges of the release layer, the adhesive layer, and the adhesive support layer are all outside the outer edges of both the drug layer and the drug support layer; wherein the portion surrounded by the outer edges of the drug layer and the drug support layer, and the inner sides of the release layer and the adhesive layer has a space; and wherein the cross-sectional area of the space is 0.3 mm² or more, at least when cut along the longitudinal centerline and the transverse centerline on the plane surface of the patch. Hereinafter, the present invention patch will be described in detail.

### 1.1 Release Layer

The release layer according to the present invention is directed mainly to protect the below-mentioned drug layer or adhesive surface until use. The release layer is usually a film made from paper or plastic film, or a sheet or a laminated film made from plastic. Specific examples of the release layer can include those composed of polyester resins such as polyethylene terephthalate (PET), polybutylene terephthalate and the like, or polyolefin resins such as polyethylene, polypropylene, and the like, and may be a laminated film obtained by laminating these resins with metals such as aluminum and other resins. In the case that paper is used for the release layer, treatment with some release agents is usually necessary. Examples of the release agents can include silicone, and siliconized paper or the like in which paper is treated with a silicone release agent is preferably used.

Among the above mentioned, a release layer composed of a polyester resin is preferred, and a release layer composed of PET is more preferred. Moreover, it is preferable that the silicone treatment is performed in order to facilitate peeling.

The release layer may be one sheet or a plurality of sheets which are superimposed together while being usually shifted. Moreover, it is preferable for the release layer to have slits in order to be easily peeled off at the time of use.

The plane area of the release layer is in the same size as, or larger than that of the adhesive support layer, mentioned later, of the present invention patch, and is preferably in the same size as the plane area of the adhesive support layer. If the area is the same, it is preferable that the shape is also the same as that of the adhesive support layer. The plane area of the release layer is also in the same size as, or larger than the plane area of the adhesive layer, mentioned later, and is preferably in the same size as the plane area of the adhesive layer.

Here, the plane area, or size of the release layer refers to the area on the inner side of the outermost edge when viewed from the direction perpendicular to the release layer surface in the case where a cut is formed or a plurality of sheets are shifted.

### 1.2 Drug Layer

The drug layer according to the present invention is directed to retain a drug. It usually has an adhesive force, and is to be peeled off from the above-mentioned release layer and adhered to the skin at the time of use to allow the retained drug to penetrate.

The drug to be retained in the drug layer is not particularly limited as long as it is a pharmacologically active ingredient and can be applied topically or systemically, and examples thereof can include basic drugs and acidic drugs. Basic drugs are preferred. Drugs having an amino group are particularly preferred.

Specific examples of the basic drugs can include skeletal muscle relaxants such as tizanidine and the like; opioid analgesics such as oxycodone, hydromorphone, fentanyl and the like; local anesthetics such as lidocaine and the like; and anti-Alzheimer agents such as donepezil and the like. While the effect of the present invention can be exerted in any of the drugs, skeletal muscle relaxants and opioid analgesics are particularly preferable, and among these, skeletal muscle relaxants are more preferable.

Examples of the skeletal muscle relaxants can include tizanidine, eperisone, and the like, and tizanidine is more preferred.

Moreover, in the case of the drug layer containing colored drugs, it is concerned that conventional patches allow the drug to diffuse into the adhesive layer or the like, causing an impairment of the appearance at the time of use. On the other hand, the present invention patch allows the drug to be uniformly spread on the drug layer and to be retained in the drug layer without diffusing into the adhesive layer or the like. Thus, the appearance at the time of being produced can be kept even in use, and the aesthetic appearance may also remain good.

The drug layer may contain any components other than the drug, and can contain, for example, solvents, polymers, transdermal absorption promoters, antioxidants, and fillers.

The solvents are used primarily to increase the miscibility of other ingredients, and both hydrophilic and hydrophobic solvents can be used. The preferred hydrophilic solvents can include, for example, polyhydric alcohols and water, and the preferred hydrophobic solvents can include, for example, hydrocarbons and esters.

The preferred polyhydric alcohols can include, for example, glycerin, propylene glycol, and 1,3-butanediol.

The preferred hydrocarbons can include, for example, heptane, toluene, and liquid paraffin. The preferred esters can include, for example, ethyl acetate, diethyl sebacate, and isopropyl myristate.

The content of the solvents is appropriately determined, and is usually within the range of 5% to 60% by weight relative to the total amount in the drug layer. In particular, the content of the hydrophilic solvents is usually within the range of 5% to 20% by weight relative to the total amount in the drug layer. For the hydrophobic solvents, the content thereof is usually within the range of 5% to 40% by weight relative to the total amount in the drug layer. Moreover, the content of the solvents is usually within the range of 30 parts to 200 parts by weight per 100 parts by weight of polymers.

The polymers are mainly used to impart adhesiveness, and usually consists of a base and a tackifier. The base may be used alone, and preferably in combination with the tackifier.

Examples of the base can include a rubber-based polymer, an acrylic polymer, and a silicone-based polymer.

Examples of the rubber-based polymer can include a styrene-isoprene-styrene block copolymer and a styrene-butadiene-styrene block copolymer. Among them, the styrene-isoprene-styrene block copolymer is preferred.

Examples of the tackifier resin can include a terpene resin, rosin, and an alicyclic saturated hydrocarbon resin. Among them, the terpene resin is preferred.

The content of the polymer is appropriately determined, and is usually within the range of 30% to 70% by weight relative to the total amount in the drug layer. Especially for the base, it is usually within the range of 10% to 20% by weight relative to the total amount in the drug layer. For tackifier resins, it is usually within the range of 20% to 40% by weight relative to the total amount in the drug layer.

Examples of the transdermal absorption promoters can include fatty acids, higher alcohols, esters, organic amines, and organic acid salts. Examples of the fatty acids can include oleic acid, and oleic acid is preferred. Examples of the higher alcohols can include oleyl alcohol, and oleyl alcohol is preferred. Examples of the organic acid salts can include sorbate, and sorbate is preferred. These may be used singly or in any combination of two or more kinds. The preferred organic amines can include diisopropanolamine, triethanolamine and salts thereof.

The content of the transdermal absorption promoters is appropriately determined, and is usually within the range of 5% to 20% by weight relative to the total amount in the drug layer.

Examples of the antioxidants can include water-soluble antioxidants and hydrophobic antioxidants. Examples of the water-soluble antioxidants can include sulfites, and among them, sodium sulfite is preferred. Examples of the hydrophobic antioxidants can include gallic acid esters, and propyl gallate is preferred. These may be used singly or in any combination of two or more kinds.

The content of the antioxidants is appropriately determined, and is usually within the range of 0.01% to 1% by weight relative to the total amount in the drug layer.

Examples of the fillers can include light anhydrous silicic acid, and light anhydrous silicic acid is preferred. The fillers may be used singly or in any combination of two or more kinds.

The content of the fillers is appropriately determined, and is usually within the range of 0.5% to 5% by weight relative to the total amount in the drug layer.

The plane area of the drug layer is appropriately determined, and is suitably within the range of 90% to 100% of the plane area of the drug support layer mentioned later, and preferably both areas are the same size. In the case of the same area size, the same shape is more preferable. The plane area of the drug layer is suitably within the range of 20% to 90% of the plane area of the adhesive layer mentioned later. It is preferable that the drug layer does not protrude from the adhesive layer.

The shape of the drug layer is not particularly limited, and examples thereof can include a quadrangle such as a square, a rectangle, and the like; a polygon, a round, and an ellipse. Among them, a quadrangle is preferable, and a rectangle is more preferable. Among the quadrangles, a rounded quadrangle is preferable, and a rounded rectangle is more preferable.

### 1.3 Drug Support Layer

The drug support layer according to the present invention is directed to support the drug layer, to cover an upper surface of the drug layer, and to separate the drug layer and the adhesive layer. The drug support layer has elasticity. In the present invention, the phrase "has elasticity" refers to a state such that when a sheet of a material used for the drug support layer, which is cut into 5 cm × 30 cm, is fixed at 1 cm inside from a corner of the sheet and at 1 cm inside from the diagonal corner of the sheet, followed by pulling at 14.7 N, the distance between the fixed portions is extended to become 110% or more, and when the pulling force is released, the distance contracts to 105% or less. That is, what is meant is an expansion or shrinkage of 10% or more.

Since the drug support layer has elasticity, it is usually composed of a material having elasticity. Examples of the material having such elasticity can include a nonwoven fabric, a woven fabric, and an elastomer film. Combinations of these may also be used.

The drug support layer may be composed of one kind or two or more kinds of materials. Among these, those composed of two kinds of materials, such as a nonwoven fabric or a PET film/nonwoven fabric laminate, are suitable.

For nonwoven fabrics, it is suitable that the weight per unit is within the range of 50 g/m²to 160 g/m², preferably within the range of 70 g/m² to 120 g/m², and more preferably within the range of 80 g/m² to 110 g/m².

As the nonwoven fabric, for example, it is preferably composed of any one of polyester resins, polyolefin resins, and cotton, or a combination thereof, more preferably composed of polyester resins, and still more preferably composed of PET.

The thickness of the drug support layer is appropriately determined, and the average thickness is preferably 0.4 mm or more. The thickness of 0.4 mm or more is expected to bring about a sufficient space around the drug layer leading to an increase of drug retention, and also to increase the stress of the adhesive layer toward the skin leading to an improved adhesiveness. It is more preferable that an average thickness is 0.5 mm or more, and even more preferably 0.6 mm or more. The upper limit is usually 2.0 mm or 1.5 mm.

Moreover, a plurality of sheets may be overlapped with each other, and the drug support layer is preferably composed of two or less sheets, and more preferably composed of one sheet.

The plane area of the drug support layer is appropriately determined, and is suitably within the range of 20% to 90% of the plane area of the adhesive layer mentioned later. It is preferable that the drug support layer does not protrude from the adhesive layer. The shape of the drug support layer is not particularly limited, and can include a similar shape to that of the drug layer, and is preferably the same as that of the drug layer.

Moreover, the distance from the outer edge of the drug support layer to the outer edge of the adhesive layer is appropriately determined, and it is preferable that the average value of the distances is within the range of 0.5 cm to 3 cm.

### 1.4 Adhesive Layer

The adhesive layer according to the present invention is directed to enable fixation and retention of the present invention patch on the skin. The adhesive layer has an adhesive force, and penetrates a drug in the drug layer into the skin, and preferably adheres to the skin at the time of use to prevent slippage.

In the adhesive layer, a polymer for imparting an adhesive force is contained. Such polymers can include, for example, those similar to those mentioned in the section "1.2 Drug Layer" mentioned above.

The polymer usually consists of a base and a tackifier. A base may be used alone, and a tackifier may be used alone, and these may be used in combination.

Examples of the base can include a rubber-based polymer, an acrylic polymer, and a silicone-based polymer and the acrylic polymer is preferred.

Examples of the rubber-based polymer can include a styrene-isoprene-styrene block copolymer and a styrene-butadiene-styrene block copolymer. Among them, the styrene-isoprene-styrene block copolymer is preferred.

Examples of the tackifier resin can include a terpene resin, rosin, and alicyclic saturated hydrocarbon resins. Among them, the terpene resin is preferred.

The content of the polymers is appropriately determined, and is usually within the range of 70% to 100% by weight relative to the total amount in the adhesive layer.

The adhesive layer may contain any components other than a polymer, and may contain, for example, solvents, transdermal absorption promoters, antioxidants, and fillers. Specific examples and the like are the same as those mentioned in the section of the drug layer.

The plane area of the adhesive layer is appropriately determined, and is suitably within the range of 90% to 100% of the plane area of the adhesive support layer mentioned later, and preferably both areas are the same size. In this case, the same shape is more preferable.

The shape of the adhesive layer is not particularly limited, and examples thereof can include a quadrangle such as a square, a rectangle, and the like; a polygon, a round, and an ellipse. Among these, a quadrangle and a round are preferable. Moreover, it is preferable for the adhesive layer to have the same shape as that of the adhesive support layer mentioned later.

### 1.5 Adhesive Support Layer

The adhesive support layer is directed to support the adhesive layer, and to cover the adhesive layer, the drug support layer, and the drug layer.

The adhesive support layer preferably has elasticity. With the adhesive support layer having the elasticity, the elasticity of the drug support layer can be drawn out more effectively, thereby enhancing the adhesiveness to the skin and enabling efficient administration of the drug.

The adhesive support layer is preferably composed of a material having elasticity, and examples of the material having such elasticity include a nonwoven fabric, a woven fabric, and an elastomer film. Combinations of these may also be used. It is preferable that the adhesive support layer is composed of the same material as that of the drug support layer in order to develop a preferable stretching behavior. Among them, a nonwoven fabric is preferred.

For the nonwoven fabric, it is suitable that the weight per unit is within the range of 50 g/m² to 160 g/m², preferably within the range of 70 g/m² to 120 g/m², and more preferably within the range of 80 g/m² to 110 g/m².

The nonwoven fabric is preferably composed of any of polyester resins, polyolefin resins, and cotton, for example, or a combination thereof, more preferably polyester resins, and still more preferably PET.

In addition, although the adhesive support layer may be composed of a plurality of superposed sheets, the adhesive support layer is preferably composed of two or less sheets, and more preferably composed of one sheet.

The thickness of the adhesive support layer is appropriately determined, and is preferably 0.4 mm or more on average, and more preferably 0.5 mm or more. The upper limit is usually 2 mm to 1.5 mm.

1.6 Space formed by the release layer, the drug layer, the drug support layer, and the adhesive layer

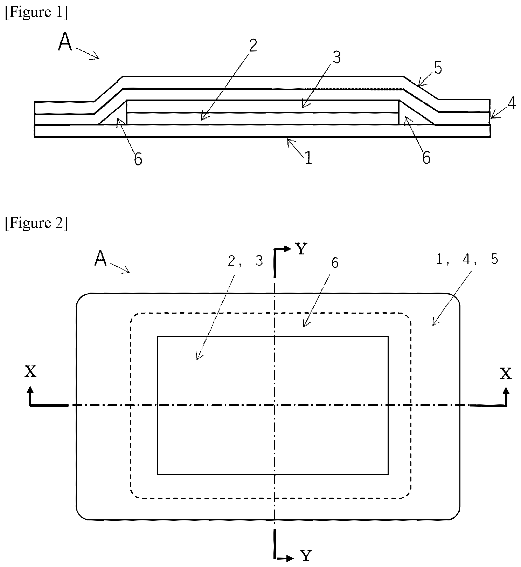

The space formed by the release layer, the drug layer, the drug support layer, and the adhesive layer (hereinafter also referred to as "the space of the present invention") is formed in a part surrounded by the outer edges of the drug layer and the drug support layer, and the inner sides of the release layer and the adhesive layer, and the cross-sectional area of the space when cut along at least the longitudinal centerline (X-X line in Figure 2) and the transverse centerline (Y-Y line in Figure 2) on the plane surface of the drug layer and the drug support layer is more than 0.3 mm². The space of the present invention is one of the important features of the present invention.

The cross-sectional area of the space of the present invention is 0.3 mm² or more, and 0.4 mm² or more is preferred, and 0. 5 mm² or more is more preferred. A preferred upper limit is, for example, 4 mm² or 3 mm².

When cutting is made in the longitudinal direction and the transverse direction on the plane surface of the drug layer and the drug support layer, the cross-sectional area of the space differs depending on the cutting site, but is 0.3 mm² or more on average including the cross-sectional area of the space under the longitudinal centerline and the transverse centerline, and 0.4 mm² or more is preferable, and 0.5 mm² or more is more preferable. A preferred upper limit is, for example, 4 mm² or 3 mm² on average.

It is preferable that the cross-sectional area of the smallest space is larger than 0 mm², but if only just a few, there may be portions where the space is 0 mm². Such portions can include, for example, a portion where the drug layer and the drug support layer are quadrangle and each corner portion thereof, i.e., the portion at which the release layer, the drug layer, the drug support layer, and the adhesive layer are all in contact at one point.

A method of adjusting the space of the present invention to a predetermined size can include, for example, a method in which the thickness of the drug support layer is adjusted, a method in which the thickness of the drug layer is adjusted, and a method in which the adhesive portions of the adhesive layer and the release layer are set distant from the drug support layer as far as possible. Among them, a method to adjust the thickness of the drug support layer is preferable because the size and shape of the space can be adjusted easily and efficiently.

### 2 Method for producing the present invention patch

The production of the present invention patch can be carried out by a conventional method, and there is no particular limitation, and examples of a suitable production method can include the following.

First, a component and a volatile solvent contained in the drug layer are mixed and coated on a surface of a release sheet. Here, examples of the volatile solvents can include ester solvents such as ethyl acetate and the like; and hydrocarbon solvents such as toluene, heptane, and the like. Among them, the ester solvents are preferred, and ethyl acetate is more preferred. The release sheet can include a material listed in the section of the release layer, and thickness or the like thereof is appropriately determined so as to be suitable for a manufacturing machine or the like.

The volatile solvent is then removed. The removal can be performed by heating, reduced pressure, or the like.

Subsequently, thereon is the drug support layer laminated.

On the other hand, the adhesive layer and the adhesive support layer are also produced by the similar method to that of the drug layer and the drug support layer. In other words, first, the above-mentioned component and volatile solvent contained in the adhesive layer are mixed and coated on the surface of the release sheet, the volatile solvent is removed, and the adhesive support layer is laminated.

The volatile solvent is suitably selected depending on the type of polymer used, and examples thereof can include an ester solvent such as ethyl acetate and the like and a hydrocarbon solvent such as toluene, heptane, and the like. Among them, an ester solvent is preferred, and ethyl acetate is more preferred.

Next, the drug layer and the drug support layer are cut into a predetermined size, and bonded with a pressure on the adhesive layer after the release sheet thereon is peeled off. Here, the release sheet of the drug layer may be peeled off and the release layer may be replaced, and it is preferable that the release layer is replaced.

Subsequently, the adhesive layer and the adhesive support layer are cut to a predetermined size. In the case that the release layer has the same size as that of the adhesive support layer, they may be cut at the same time, and it is preferable to cut at the same time.

In the way as mentioned above, the present invention patch can be produced.

### EXAMPLE

Hereinafter, the present invention will be illustrated in more detail with reference to examples. The present invention is not limited to the following examples.

### Example 1 and Comparative Example 1: Production of Patch

### (1) Production of drug layer and drug support layer

The drug layer components shown in Table 1 or Table 2 (numerical value is wt%) and the volatile solvent were sequentially added into a container made of stainless steel, and sufficiently stirred to obtain a drug layer coating liquid. Next, the drug layer coating liquid was coated on a release sheet made of PET. After removing the volatile solvent, the drug support layer shown in Table 1 or 2 was laminated to the drug layer side. Thereafter, it was cut into a rectangle of 5.5 cm × 7.3 cm.

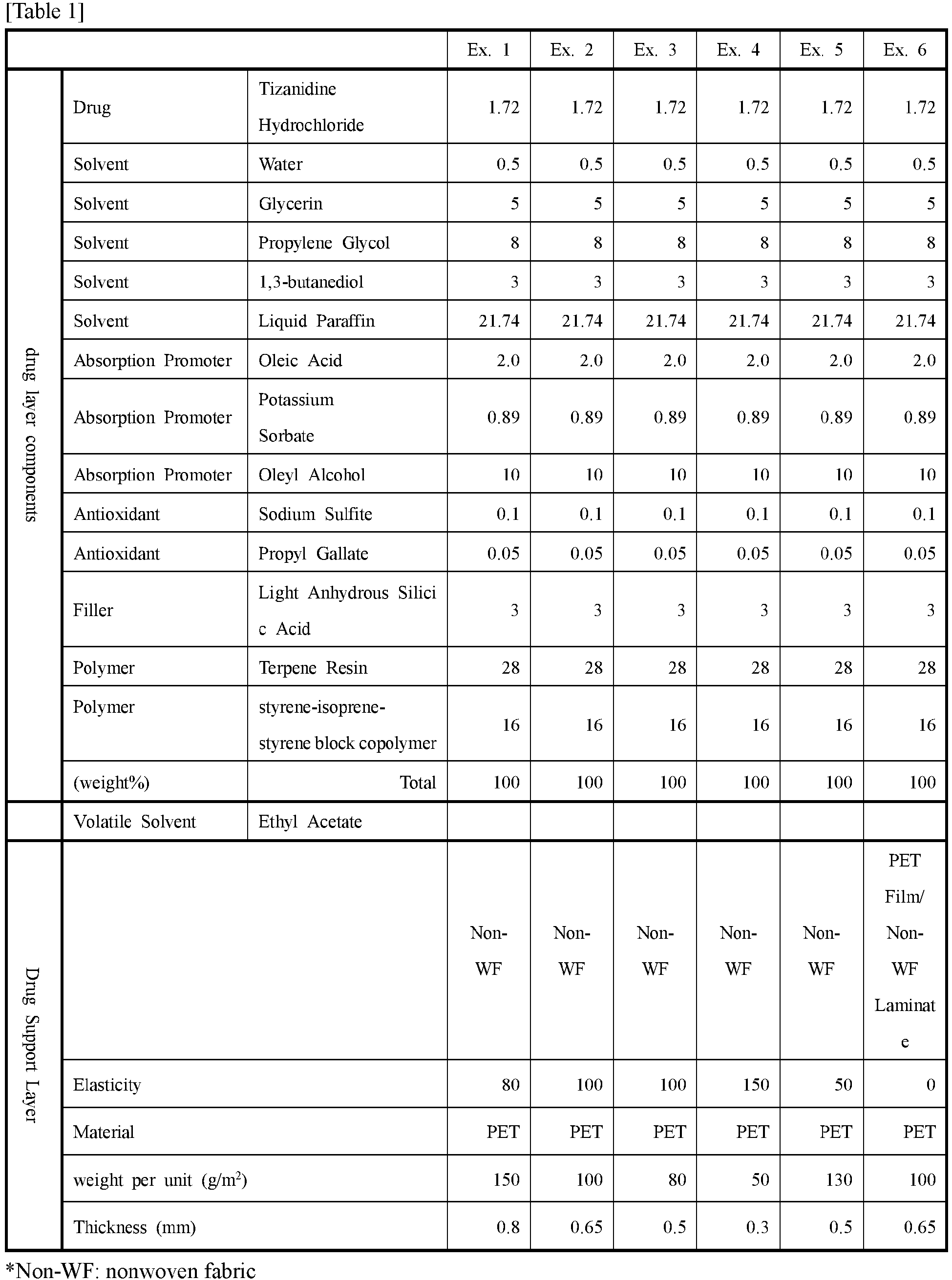

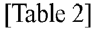

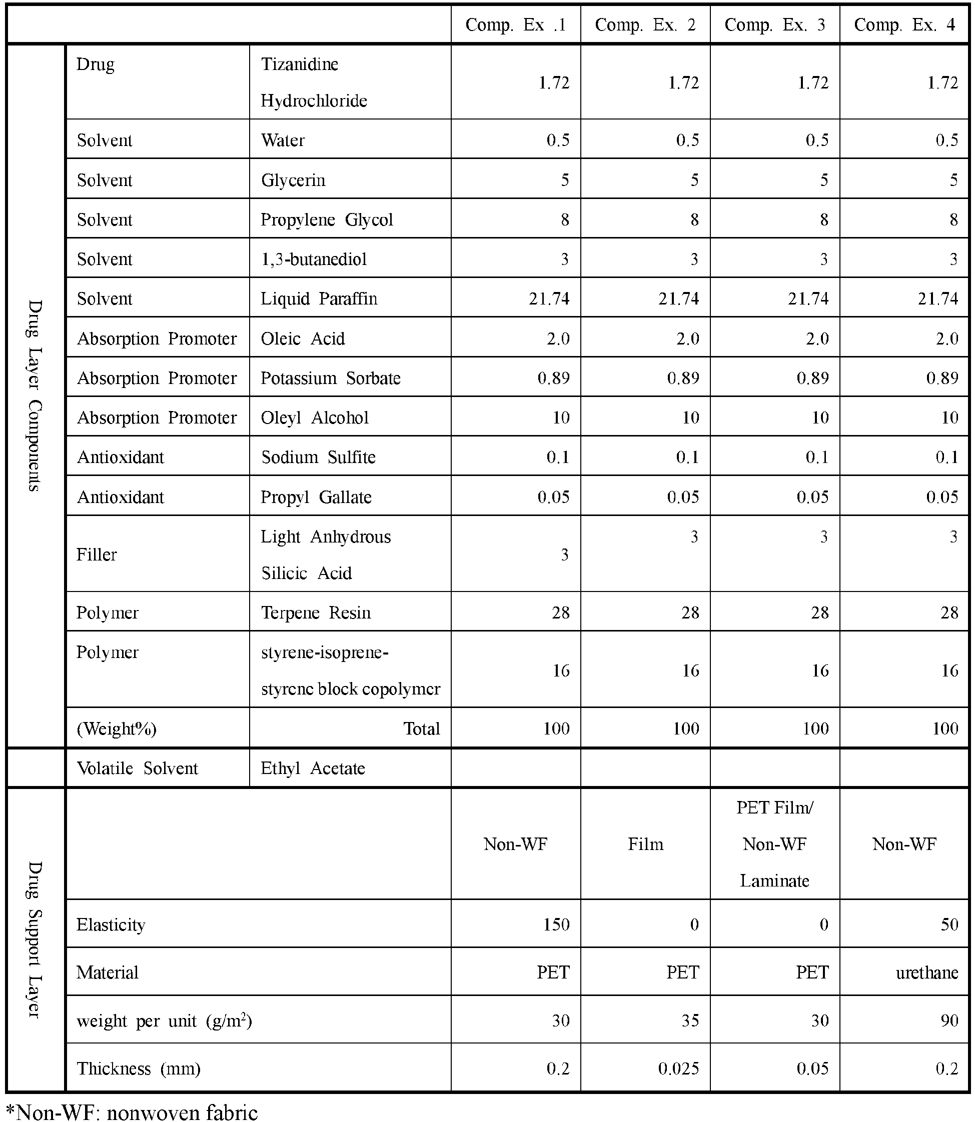

### (2) Production of adhesive layer and adhesive support layer

A release sheet made of PET was installed on a coating machine, and the adhesive layer coating liquid containing a volatile solvent shown in Table 3 was coated on the release sheet. After removing the volatile solvent, the adhesive support layer shown in Table 3 was laminated to the adhesive layer side.

**[Table 3]**

| Adhesive Layer | Acrylic Polymer | 100 weight% |
|---|---|---|
| Total | | 100 weight% |
| Volatile Solvent | Ethyl Acetate | |
| Adhesive Support Layer | Non-WF (weight per unit: 90 g/m²) | |

### (3) Production of patch

The release sheet of the adhesive layer and the adhesive support layer obtained in the (2) mentioned above was peeled off, the drug layer and the drug layer support layer obtained in the (1) mentioned above was bonded from the side of the drug layer support layer with a pressure, the release sheet on the drug layer side was peeled off, the release layer was bonded with a pressure on the drug layer side, and then the release layer was cut into a rectangle of 7.0 cm × 8.8 cm with slightly rounded corners.

### Test Example 1: Amount of drug transferred from the drug layer to the adhesive layer

With regard to Comparative Example 3 in which the drug support layer is composed of only a film and has very little space in a section surrounded by the outer edges of the drug layer and the drug support layer, and the inner sides of the release layer and the adhesive layer, Example 1 in which the drug support layer is composed of only a nonwoven fabric and has the above-mentioned space, and Example 6 in which the drug support layer is composed of a film and a nonwoven fabric and has the above-mentioned space; the adhesive layer was peeled off after storage at 40 °C for three months, and the drug component was extracted, followed by measuring the amount of transfer to the adhesive layer by liquid chromatography.

**[Table 4]**

| | | | | |
|---|---|---|---|---|
| Amount of Transfer to the Adhesive Layer (40 °C for three months) | | Comp. Ex. 3 | Ex. 1 | Ex. 6 |
| | Tizanidine | 1.6% | 0.4% | 0% |
| | Sorbic Acid | 6.9% | 12.3% | 6.3% |

Although the effect of retaining volatile sorbic acid in the drug layer was recognized when the film was used for the drug support layer, when there was no space, nonvolatile tizanidine was transferred to the adhesive layer. The PET film/nonwoven fabric laminate kept sorbic acid and tizanidine in the drug layer, and only a little amount of transfer to the adhesive layer was observed.

### Test Example 2: Adhesiveness

The patch was placed on the skin of the elbow and observed for slippage after 30 bending motions. Each symbol indicating the state of slippage refers as follows. The mark "++": No slippage was observed (no problem in use). The mark "+": Little slippage was observed. (No problem in use). The mark "±": Slippage was observed, but peeling of the patch was not observed (no problem in use). The mark "-": Slippage was observed, and a part of the patch peeled off.

### Test Example 3: Appearance

After storage at 40 °C conditions, the patch was observed from the release layer direction. When the chemical seeps out, coloration is observed from the outer peripheral portion of the drug layer to the outer side of the adhesive layer. Symbols indicating the state of coloration refers as follows. The mark "++": No coloration was observed on the outer adhesive layer side, the mark "+": Slight coloration was observed on the outer adhesive layer side, the mark "-": Coloration was observed on the outer adhesive layer side.

The spatial cut section in Table 5 was the cross-sectional area of the space when cut along the longitudinal centerline (X-X line in Figure 2) and the transverse centerline (Y-Y line in Figure 2) on the plane surface of the drug layer and the drug support layer, and the cross-sectional area of the space when cut in the longitudinal direction and the transverse direction except for the longitudinal centerline and the transverse centerline was substantially the same as the spatial cut section mentioned above.

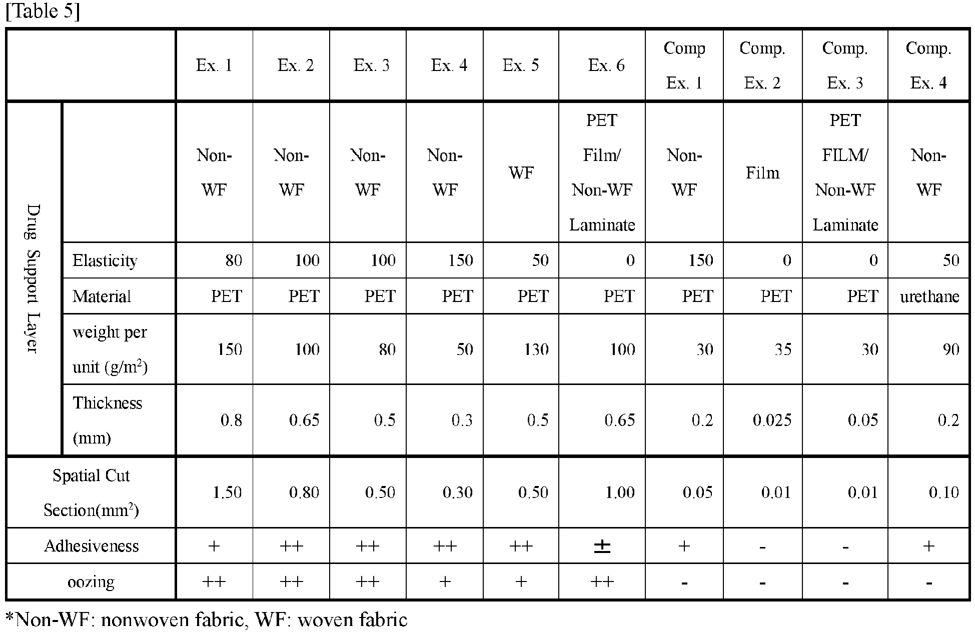

As is apparent from the results shown in Table 4 and Table 5, the present invention patch is excellent in the residual amount of the drug after storage and the appearance, and is also excellent in adhesiveness to the skin.

### INDUSTRIAL APPLICABILITY

Since the present invention patch can keep a drug even after storage and is also excellent in adhesiveness to the skin, it is useful as a pharmaceutical or the like.

### DESCRIPTION OF SYMBOLS

- A: Present invention patch
- 1: Release layer
- 2: Drug layer
- 3: Drug support layer
- 4: Adhesive layer
- 5: Adhesive support layer
- 6: Space formed by the release layer, the drug layer, the drug support layer, and the adhesive layer

## Claims

1. A laminated type patch, comprising a release layer, a drug layer, a drug support layer having elasticity, an adhesive layer, and an adhesive support layer laminated in this order,
wherein the outer edges of the release layer, the adhesive layer, and the adhesive support layer are all outside the outer edges of both the drug layer and the drug support layer;
wherein the portion surrounded by the outer edges of the drug layer and the drug support layer, and the inner sides of the release layer and the adhesive layer has a space; and
wherein the cross-sectional area of the space is 0.3 mm² or more, at least when cut along the longitudinal centerline and the transverse centerline on the plane surfaces of the drug layer and the drug support layer.

2. The laminated type patch according to Claim 1, wherein the cross-sectional area of the space is 0.3 mm² or more on average, when cut in the longitudinal direction and the transverse direction including the longitudinal centerline and the transverse centerline on the plane surfaces of the drug layer and the drug support layer.

3. The laminated type patch according to Claim 1 or 2, wherein the average thickness of the drug support layer is 0.4 mm or more.

4. The laminated type patch according to any one of Claims 1 to 3, wherein the drug support layer consists of one or more kinds selected from the group consisting of a nonwoven fabric, a woven fabric, and an elastomer film.

5. The laminated type patch according to Claim 4, wherein the drug support layer consists of a nonwoven fabric.

6. The laminated type patch according to Claim 5, wherein the weight per unit of the nonwoven fabric is within the range of 50 to 160 g/m².

7. The laminated type patch according to Claim 5 or 6, wherein the nonwoven fabric consists of a polyester resin.

8. The laminated type patch according to any one of Claims 1 to 7, wherein the adhesive support layer has elasticity.

9. The laminated type patch according to any one of Claims 1 to 8, wherein the drug layer contains a colored drug.

10. The laminated type patch according to any one of Claims 1 to 9, wherein the drug layer contains one or more solvents selected from the group consisting of a hydrocarbon, a polyhydric alcohol, and an ester.

11. The laminated type patch according to any one of Claims 1 to 10, wherein the adhesive layer contains one or more polymers selected from the group consisting of an acrylic polymer, a rubber-based polymer, and a silicone-based polymer.

12. The laminated type patch according to any one of Claims 1 to 11, wherein the release layer is treated with a silicone.

13. The laminated type patch of any one of Claims 1 to 12, wherein the plane area of the drug layer is within the range of 90% to 100% of the plane area of the drug support layer.

14. The laminated type patch according to any one of Claims 1 to 13, wherein the plane area of the drug layer is within the range of 20% to 90% of the plane area of the adhesive layer.

15. The laminated type patch according to any one of Claims 1 to 14, wherein the plane area of the drug support layer is within the range of 20% to 90% of the plane area of the adhesive layer.

16. The laminated type patch according to any one of Claims 1 to 15, wherein the average value of a distance from an outer edge of the drug support layer to an outer edge of the adhesive layer is within the range of 0.5 cm to 3 cm.

17. The laminated type patch according to any one of Claims 1 to 16, wherein the plane area of the adhesive layer is within the range of 90% to 100% of the plane area of the adhesive support layer.
